# EUROPEAN PATENT APPLICATION

(11) **EP 3 072 894 A1**
(43) Date of publication of application: **28.09.2016**
(21) Application number: 15161153.0
(22) Date of filing: 26.03.2015
(51) Int. Cl.: C07D 487/04, A61K 31/519, A61P 33/06

(54) **NEW SUBSTITUTED TRIAZOLOPYRIMIDINES AS ANTI-MALARIAL AGENTS**

(71) Applicant: Board Of Regents Of the University Of Texas System, Austin, TX 78701 (US); Monash University, Clayton, Victoria 3800 (AU); University of Washington through its Center for Commercialization, Seattle, WA 98105 (US); MMV Medicines for Malaria Venture, 1215 Geneva (CH)
(72) Inventor: Phillips, Margaret, Dallas, TX 75214 (US); Charman, Susan A., South Melbourne, Victoria 3205 (AU); Rathod, Pradipsinh K., Seattle, WA 98103 (US); Matthews, David, Encinitas, CA 92024 (US); Waterson, David, Macclesfield, Cheshire SK10 3JQ (GB)
(74) Representative: reuteler & cie SA

(57) **Abstract**

The present invention is related to a use of triazolopyrimidine derivatives in the manufacture of a medicament for preventing or treating malaria. Specifically, the present invention is related to triazolopyrimidine derivatives useful for the preparation of a pharmaceutical formulation for the inhibition of malaria parasite proliferation.

## Description

### Field of the Invention

The present invention relates to novel anti-malarial agents. Specifically, the present invention is related to agents useful for the preparation of a pharmaceutical formulation for preventing or treating malaria and methods of their use and manufacture.

### Background of the Invention

Malaria is caused by protozoan parasites of the genus *Plasmodium* that infect and destroy red blood cells, leading to fever, severe anemia, cerebral malaria and, if untreated, death. *Plasmodium falciparum* is the dominant species in sub-Saharan Africa, and is responsible for approximately 600,000 deaths each year. The disease burden is heaviest in African children under 5 years of age and in pregnant women. *Plasmodium vivax* causes 25-40% of the global malaria burden, particularly in South and Southeast Asia, and Central and South America. The other three main species that are known to infect humans are *Plasmodium ovale, Plasmodium knowelsi* and *Plasmodium malariae.*

Malaria is a disease that is prevalent in many developing countries. Approximately 40% of the world's population lives in countries where the disease is endemic; approximately 247 million people suffer from the disease every year.

Various medications are presently used for the treatment of malaria. However, many of these medications are costly and some exhibit significant toxicity and undesirable side effects in humans. Drugs used for treating malaria include artemisinin and its derivatives (such as artemether or dihydroartemisinin, chloroquine, quinine, mefloquine, amodiaquine, atovaquone/proguanil, doxycycline, lumefantrine, piperaquine, pyronaridine, halofantrine, pyrimethamine-sulfadoxine, primaquine, quinacrine, doxycycline, atovaquone, proguanil hydrochloride, piperaquine, ferroquine, tafenoquine, arterolane, Spiro[3H-indole-3,1'-[1H]pyrido[3,4-b]indol]-2(1H)-one, 5,7'-dichloro-6'-fluoro-2',3',4',9'-tetrahydro-3'-methyl-,(1'R,3'S)-] (CAS Registry Number: 1193314-23-6), Sulfur, [4-[[2-(1,1-difluoroethyl)-5-methyl[1,2,4]triazolo[1,5-a]pyrimidin-7-yl]amino]phenyl] pentafluoro-] (CAS Registry Number: 1282041-94-4), Morpholine, 4-[2-(4-cis-dispiro[cyclohexane-1,3'-[1,2,4] trioxolane-5',2"-tricyclo[3.3.1.13,7]decan]-4-ylphenoxy)ethyl]-] (CAS Registry Number: 1029939-86-3).

However, the widespread emergence of drug resistance of malaria parasites in many tropical countries has compromised many of the current chemotherapies and there is a continued need for new chemotherapeutic approaches.

*P. falciparum* is transmitted to humans via the bite of an infected female anopheline mosquito. In humans, the parasite undergoes one cycle of asexual multiplication in hepatocytes, followed by several cycles of infection and multiplication in red blood cells. If the hepatocytic stage is asymptomatic, the erythrocytic stage comprises the destruction of the host erythrocytes, resulting in anemia leading to death, in absence of treatment. Purine metabolism holds significant promise as a target for drug development.

It has long been recognized that *Plasmodium* parasites lack the ability to metabolize exogenous pyrimidines and instead are entirely dependent on *de novo* pyrimidine biosynthesis to provide precursors for DNA and RNA synthesis, and hence for proliferation. The parasite does not have pyrimidine nucleoside or base salvage pathways, thus the enzymes in the *de novo* pathway are essential to parasite survival. In contrast, mammalian cells have salvage pathways that provide an alternative route to these essential metabolites.

Dihydroorotate dehydrogenase (DHODH) is an essential enzyme of the pyrimidine salvage pathway, and a number of studies suggest that it is an important target for the development of new chemotherapy against malaria. DHODH is a flavin-dependent mitochondrial enzyme that catalyzes the flavin mononucleotide (FMN)-dependent oxidation of dihydroorotate to orotic acid, an essential step in *de novo* pyrimidine biosynthesis. Both human and malaria DHODH are mitochondrial enzymes, but X-ray structural analysis has shown that if the overall fold is well-conserved, the presumptive CoQ binding site is variable between species. An inhibitor of human DHODH (HsDHODH) (teriflunomide (A77 1726), the active metabolite of leflunomide is clinically approved for the treatment of rheumatoid arthritis and multiple sclerosis, and a number of compounds have been described that either bind potently to the human enzyme (e.g., brequinar and C41) or selectively inhibit DHODH from various microbial species, demonstrating that DHODH is a druggable target (Miller et al., 2013, Nat. Med., 19, 156-67*;* Munier-Lehmann et al., 2013, J. Med. Chem., 56, 3148-3167*;* Phillips et al., 2010, Infect. Discord. Drug Targets, 10, 226-239). Triazolopyrimidine and imidazo[1,2-a]pyrimidine- based inhibitors of *P. falciparum* dihydroorotate dehydrogenase that inhibit *parasite in vitro* growth with similar activity have been developed (Philips et al., 2008, J. Med. Chem., 51, 3649-3653*;* Marwaha et al., 2012, J. Med. Chem., 55, 7425-7436*;* WO 2011041304*;* Deng et al., 2014, J. Med. Chem., 57, 5381-5394*;* Coteron et al., 2011, J. Med. Chem., 54, 5540-5561)*.*

There is also a need for an antimalarial agent to overcome current drug resistance problems with existing therapy. Further, anti-malarial agents are needed that selectively inhibit malarial DHODH but exhibit no substantial toxicity against mammalian, especially human DHODH.

Accordingly, this invention provides novel potent anti-malarial agents and methodology of treating malaria using novel potent anti-malarial agents. The invention also provides potent anti-malarial agents that are selective inhibitors of *P. falciparum* dihydroorotate dehydrogenase and active against chloroquine-sensitive and resistant malarial strains.

### Summary of the Invention

The present invention is directed to the unexpected findings of triazolopyrimidines exhibiting improved selectivity for inhibition of plasmodial DHODH over mammalian DHODH and improved solubility, whilst exhibiting long *in vivo* half-life.

The present invention is directed towards novel triazolopyrimidine derivatives that are useful in the treatment and/or prophylaxis of malaria, pharmaceutical formulation, use and manufacture thereof.

A first aspect of the invention provides novel triazolopyrimidine derivatives according to the invention or a pharmaceutically acceptable salt thereof.

A second aspect of the invention relates to novel triazolopyrimidine derivatives or a pharmaceutically acceptable salt thereof according to the invention for use as a medicament. A third aspect of the invention relates to the use of novel triazolopyrimidine derivatives according to the invention or a pharmaceutically acceptable salt thereof, for the preparation of a pharmaceutical composition for the prevention and/or treatment of malaria.

A fourth aspect of the invention resides in a pharmaceutical formulation comprising at least one novel triazolopyrimidine derivative according to the invention or a pharmaceutically acceptable salt thereof, and a pharmaceutically acceptable carrier, diluent or excipient thereof.

A fifth aspect of the invention relates to novel triazolopyrimidine derivatives according to the invention or a pharmaceutically acceptable salt thereof, for use in the prevention and/or treatment of malaria.

A sixth aspect of the invention resides in a method for preventing and/or treating malaria in a patient. The method comprises administering novel triazolopyrimidine derivatives according to the invention or a pharmaceutically acceptable salt in a patient in need thereof.

A seventh aspect of the invention provides a process for the preparation of novel triazolopyrimidine derivatives according to the invention or a pharmaceutically acceptable salt thereof according to the invention and intermediates thereof.

An eighth aspect of the invention provides intermediates of synthesis of triazolopyrimidine derivatives according to the invention.

A ninth aspect of the invention provides a method for inactivating parasitic infection in a cell comprising the step of contacting the cell with an effective amount of at least one compound according to the invention.

### Detailed Description of the invention

The following paragraphs provide definitions of the various chemical moieties that make up the compounds according to the invention and are intended to apply uniformly through-out the specification and claims, unless an otherwise expressly set out definition provides a broader definition.

The term "pharmaceutically acceptable salts or complexes" refers to salts or complexes of the compounds according to the invention. Examples of such salts include, but are not restricted, to base addition salts formed by reaction of triazolopyrimidine derivatives of the invention with organic or inorganic bases such as hydroxide, carbonate or bicarbonate of a metal cation such as those selected in the group consisting of alkali metals (sodium, potassium or lithium), alkaline earth metals (e.g. calcium or magnesium).

Also comprised are salts which are formed from acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, benzene sulphonic acid, methane sulphonic acid, tannic acid, palmoic acid, alginic acid, polyglutamic acid, naphthalene sulfonic acid, naphthalene disulfonic acid, and poly-galacturonic acid.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. The prodrug is a derivative of the compounds according to the invention and presenting anti-malarial activity that has a chemically or metabolically decomposable group, and a compound that may be converted into a pharmaceutically active compound according to the invention *in vivo* by solvolysis under physiological conditions. The prodrug is converted into a compound according to the present invention by a reaction with an enzyme, gastric acid or the like under a physiological condition in the living body, e.g. by oxidation, reduction, hydrolysis or the like, each of which is carried out enzymatically. These compounds can be produced from compounds of the present invention according to well-known methods.

The term "indirectly" also encompasses metabolites of compounds according to the invention.

The term "metabolite" refers to all molecules derived from any of the compounds according to the present invention in a cell or organism, preferably mammal.

The term "malaria" includes disease and conditions related to an infection by *Plasmodium.*

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

The term "effective amount" includes "prophylaxis-effective amount" as well as "treatment-effective amount" and can refer to the amount used as part of a combination.

The term "prophylaxis-effective amount" refers to a concentration of compound of this invention that is effective in inhibiting, decreasing the likelihood of the disease by malarial parasites, or preventing malarial infection or preventing the delayed onset of the disease by malarial parasites, when administered before infection, i.e. before, during and/or slightly after the exposure period to malarial parasites.

The term "prophylaxis" includes causal prophylaxis, i.e. antimalarial activity comprising preventing the pre-erythrocytic development of the parasite, suppressive prophylaxis, i.e. antimalarial activity comprising suppressing the development of the blood stage infection and terminal prophylaxis, i.e. antimalarial activity comprising suppressing the development of intra-hepatic stage infection. This term includes primary prophylaxis (i.e. preventing initial infection) where the antimalarial compound is administered before, during and/or after the exposure period to malarial parasites and terminal prophylaxis (i.e. to prevent relapses or delayed onset of clinical symptoms of malaria) when the antimalarial compound is administered towards the end of and/or slightly after the exposure period to malarial parasites but before the clinical symptoms. Typically, against *P. falciparum* infections, suppressive phophylaxis is used whereas against *P. vivax* or a combination of *P. falciparum* and *P. vivax,* terminal prophylaxis is used. According to one embodiment, the malaria parasites are P. *falciparum* and *P. vivax.*

Likewise, the term "treatment-effective amount" refers to a concentration of compound that is effective in treating malaria infection, e.g. leads to a reduction in parasite numbers in blood following microscopic examination when administered after infection has occurred.

The term "subject" as used herein refers to mammals. For examples, mammals contemplated by the present invention include humans and the like.

### Compounds

According to one embodiment, is provided a triazolopyrimidine derivative according to Formula (I): 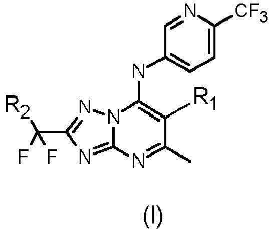 wherein R₁ is selected from halogen such as F and H, R₂ is selected from F and C₁-C₄ alkyl such as methyl; as well as any pharmaceutically acceptable salt, hydrate, solvate, polymorph, tautomers, geometrical isomers, or optically active isomers thereof.

In a particular embodiment, the invention provides a triazolopyrimidine derivative according to the invention wherein R₁ is H.

In a particular embodiment, the invention provides a triazolopyrimidine derivative according to the invention wherein R₁ is F.

In a particular embodiment, the invention provides a triazolopyrimidine derivative according to the invention wherein is R₂ is CH₃.

In a particular embodiment, the invention provides a triazolopyrimidine derivative according to the invention wherein is R₂ is F.

In a particular embodiment is provided a triazolopyrimidine derivative selected from the following group:
2-(1,1-difluoroethyl)-5-methyl-N-(6-(trifluoromethyl)pyridin-3-yl)-[1,2,4]triazolo[1,5-a] pyrimidin-7-amine;
2-(1,1-difluoroethyl)-6-fluoro-5-methyl-N-(6-(trifluoromethyl)pyridin-3-yl)-[1,2,4]triazolo [1,5-a]pyrimidin-7-amine; and
5-methyl-2-(trifluoromethyl)-N-(6-(trifluoromethyl)pyridin-3-yl)-[1,2,4]triazolo[1,5-a] pyrimidin-7-amine; as well pharmaceutically acceptable salt, hydrate, solvate, polymorph, tautomers, geometrical isomers, or optically active isomers thereof.

The triazolopyrimidine derivatives used in the manufacture of a medicament for the prevention or treatment of malaria, are capable of killing and/or inhibiting malaria parasite replication and/or blocking transmission.

### Compositions

The invention provides pharmaceutical compositions useful for the prophylaxis and/ or treatment of malaria. The invention further provides methods for treating a mammalian patient, and most preferably a human patient, who is suffering from malaria.

In another particular embodiment, is provided a pharmaceutical formulation containing at least one derivative according to the invention and a pharmaceutically acceptable carrier, diluent or excipient thereof.

In another particular embodiment, is provided a pharmaceutical formulation comprising a triazolopyrimidine according to Formula (I) and an antimalarial agent as defined in the detailed description.

Pharmaceutical compositions of the invention can contain one or more compound(s) of the invention in any form described herein. Compositions of this invention may further comprise one or more pharmaceutically acceptable additional ingredient(s), such as alum, stabilizers, antimicrobial agents, buffers, coloring agents, flavoring agents, adjuvants, and the like.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended dosage range to be employed. Compositions according to the invention are preferably oral.

Compositions of this invention may be liquid formulations, including, but not limited to, aqueous or oily suspensions, solutions, emulsions, syrups, and elixirs. Liquid forms suitable for oral administration may include a suitable aqueous or non-aqueous vehicle with buffers, suspending and dispensing agents, colorants, flavors and the like. The compositions may also be formulated as a dry product for reconstitution with water or other suitable vehicle before use. Such liquid preparations may contain additives, including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Non-aqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid. Further materials as well as processing techniques and the like are set out in The Science and Practice of Pharmacy (Remington: The Science & Practice of Pharmacy), 22nd Edition, 2012, Lloyd, Ed. Allen, Pharmaceutical Press*,* which is incorporated herein by reference.

Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate. Tablets may be coated according to methods well known in the art. Injectable compositions are typically based upon injectable sterile saline or phosphate-buffered saline or other injectable carriers known in the art.

Compositions of this invention may also be formulated as suppositories, which may contain suppository bases including, but not limited to, cocoa butter or glycerides. Compositions of this invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of this invention may also be formulated transdermal formulations comprising aqueous or non-aqueous vehicles including, but not limited to, creams, ointments, lotions, pastes, medicated plaster, patch, or membrane.

Compositions of this invention may also be formulated for parenteral administration, including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water. Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated as a liposome preparation. The liposome preparation can comprise liposomes which penetrate the cells of interest or the *stratum corneum,* and fuse with the cell membrane, resulting in delivery of the contents of the liposome into the cell. Other suitable formulations can employ niosomes. Niosomes are lipid vesicles similar to liposomes, with membranes consisting largely of non-ionic lipids, some forms of which are effective for transporting compounds across the *stratum corneum*.

The compounds of this invention can also be administered in sustained release forms or from sustained release drug delivery systems. A description of representative sustained release materials can also be found in the incorporated materials in *Remington's Pharmaceutical*

*Sciences.*

### Mode of administration

Compositions of this invention may be administered in any manner, including, but not limited to, orally, parenterally, rectally, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion. In a preferred embodiment, triazolopyrimidine derivatives according to the invention are administered orally.

In a particular embodiment, compounds of the invention are administered at a dose to humans of between about 1 mg and 1500 mg such as for example from about 25-750 mg, such as from about 80 to about 170 mg, for example at about 150 mg. In a further particular embodiment, compound of the invention are administered at a dose of less than 500 mg.

This invention is further illustrated by the following examples that are not intended to limit the scope of the invention in any way.

The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

The compositions of this invention may be used in a method for inactivating parasitic infection in a cell comprising the step of contacting the cell with an effective amount of at least one compound according to the invention. According to a particular aspect, the cell is a primate cell such as a red blood cell for example a human cell.

### Combination

According to the invention, the triazolopyrimidine derivatives of the invention and pharmaceutical formulations thereof can be administered alone or in combination with a co-agent useful in the treatment of malaria, such as substances useful in the treatment and/or prevention of malaria e.g. for example a co-agent including, but not limited to, artemisinin or an artemisinin and its derivatives (such as artemether or dihydroartemisinin, chloroquine, quinine, mefloquine, amodiaquine, atovaquone/proguanil, doxycycline, lumefantrine, piperaquine, pyronaridine, halofantrine, pyrimethamine-sulfadoxine, primaquine, quinacrine, doxycycline, atovaquone, proguanil hydrochloride, piperaquine, ferroquine, tafenoquine, arterolane, Spiro[3H-indole-3,1'-[1H]pyrido[3,4-b]indol]-2(1H)-one, 5,7'-dichloro-6'-fluoro-2',3',4',9'-tetrahydro-3'-methyl-,(1'R,3'S)- (CAS Registry Number: 1193314-23-6), Sulfur, [4-[[2-(1,1-difluoroethyl)-5-methyl[1,2,4]triazolo[[1,5-a]pyrimidin-7-yl]amino]phenyl] pentafluoro-] (CAS Registry Number: 1282041-94-4), Morpholine,4-[2-(4-cis-dispiro [cyclohexane-1,3'-[1,2,4]trioxolane-5',2"-tricyclo[3.3.1.1^{3,7}]decan]-4-ylphenoxy)ethyl]- (CAS Registry Number: 1029939-86-3), [3,3'-Bipyridin]-2-amine, 5-[4-(methylsulfonyl)phenyl]-6'-(trifluoromethyl)- (CAS Registry Number: 1314883-11-8), Ethanone, 2-amino-1-[2-(4-fluorophenyl)-3-[(4-fluorophenyl)amino]-5,6-dihydroimidazo [1,2-a]pyrazin-7(8H)-yl]-(CAS Registry Number 1261109-90-3).

The invention encompasses the administration of a triazolopyrimidine derivative according to the invention or of a pharmaceutical formulation thereof, wherein the triazolopyrimidine derivatives or the pharmaceutical formulation thereof is administered to an individual prior to, simultaneously or sequentially with other therapeutic regimens or co-agents useful in the treatment of malaria (e.g. multiple drug regimens), in an effective amount. Triazolopyrimidine derivatives or the pharmaceutical formulations thereof that are administered simultaneously with said co-agents can be administered in the same or different composition(s) and by the same or different route(s) of administration.

### Patients

In an embodiment, patients according to the invention are patients suffering from malaria.

In another embodiment, patients according to the invention are patients with a high risk of being infected by *Plasmodium.*

In another embodiment, patients according to the invention are patients with a high risk of being infected by *Plasmodium falciparum.*

In another embodiment, patients according to the invention are patients with a high risk of being infected by *Plasmodium vivax.*

### Process of preparation

In an embodiment according to the invention, is provided a method for preparing a compound of Formula (I) wherein R₁ is F and R₂ is CH₃ comprising a step of reacting a triazolopyrimidine of Formula **(5b)** in presence of 5-amino-2-trifluoromethylpyridine as follows:

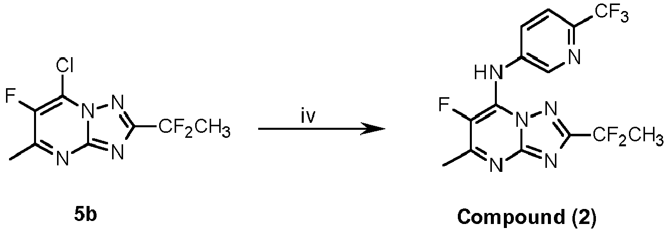

In another embodiment according to the invention, is provided a method for preparing a compound of Formula (I) wherein R₁ is F and R₂ is CH₃ comprising a step of reacting a triazolopyrimidine of Formula **(4b)** in presence of POCl₃ to lead to an intermediate of Formula (5b) as follows:

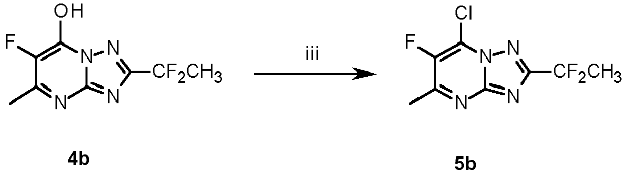

In another embodiment, is provided an intermediate for the preparation of a compound of Formula (I), wherein the intermediate is 2-(1,1-difluoroethyl)-6-fluoro-5-methyl-[1,2,4]triazolo[1,5-a]pyrimidin-7-ol.

In another embodiment, is provided an intermediate for the preparation of a compound of Formula (I), wherein the intermediate is 7-chloro-2-(1,1-difluoroethyl)-6-fluoro-5-methyl-[1,2,4]triazolo[1,5-a]pyrimidine.

### Use according to the invention

In one embodiment, the invention provides a use of a triazolopyrimidine derivative according to Formula (I) as described herein, as well pharmaceutically acceptable salt, hydrate, solvate, polymorph, tautomers, geometrical isomers, or optically active forms thereof for the preparation of a pharmaceutical composition for the treatment or prophylaxis of malaria.

In another embodiment, the invention provides a method for preventing or treating malaria in a patient. The method comprises administering an effective amount of a triazolopyrimidine derivative according to the invention, or a pharmaceutically acceptable salt or a pharmaceutically active derivative thereof or a pharmaceutical formulation thereof in a patient in need thereof.

In another embodiment, the invention provides a triazolopyrimidine derivative according to the invention as well as pharmaceutically acceptable salts or a pharmaceutically active derivative thereof or a pharmaceutical formulation thereof, for use in the treatment or prophylaxis of malaria.

In another embodiment, the invention provides a use of a triazolopyrimidine derivative or a method according to the invention wherein the triazolopyrimidine derivative is to be administered in combination with a co-agent useful in the treatment of malaria.

In another embodiment, the invention provides a pharmaceutical composition comprising a triazolopyrimidine derivative according to the invention in combination with a co-agent useful in the treatment of malaria.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description. Such modifications are intended to fall within the scope of the appended claims. In the following the present invention shall be illustrated by means of some examples, which are not to be viewed as limiting the scope of the invention.

### EXAMPLES

### The following abbreviations refer respectively to the definitions below:

**ACN** (acetonitrile), **CoQ** (Coenzyme Q), **DCIP** (2,6-dichloroindophenol), **DMSO** (Dimethyl Sulfoxide), **HCT** (hematocrit), **HEPES** (4-(2-hydroxyethyl)-1-piperazineethanesulfonic acid), **NMR** (Nuclear magnetic Resonance), **NOD** (non-obese diabetic), **PBS** (Phosphate Buffer Sulfate), **RPMI** (Roswell Park Memorial Institute), **SCID** (severe compromised immunodeficiency), **UV** (Ultraviolet).

The compounds of invention have been named according to the IUPAC standards used in the program ChemDraw® 7.0. The MS and NMR data provided in the examples described below are obtained as follows below. All reagents and intermediates whose synthesis is not described were purchased from standard commercially available sources.

### Chemistry General Methods

All reagents and starting materials were obtained from commercial suppliers and used without further purification. Ethyl acetoacetate (**1a**) and Ethyl 2-fluoroacetoacetate (**1b**) were purchased from Sigma-Aldrich, MO, USA. Aminoguanidine Hydrochloride (2) was purchased from TCI chemicals, OR, USA. Ethyl 2,2-difluoropropionate was purchased from Oakwood Products, Inc. SC, USA. 5-Amino-2-trifluoromethylpyridine was purchased from Combi-Blocks, Inc. CA, USA. For Compound 1 and Compound 2, reaction progress was monitored by thin layer chromatography (TLC) on preloaded silica gel 60 F₂₅₄ plates. Visualization was achieved with UV light and iodine vapor. Flash chromatography was carried out using prepacked Teledyne Isco Redisep™ Rf silica-gel columns as the stationary phase and analytical grade solvents as the eluent unless otherwise stated. ¹H nuclear magnetic resonance (NMR) spectra were recorded on an Avance™ 301 Bruker instrument operating at 300.10 MHz at ambient temperature. Chemical shifts are reported in parts per million (δ) and coupling constants in Hz. ¹H NMR spectra were referenced to the residual solvent peaks as internal standards (7.26 ppm for CDCl₃, 2.50 ppm for DMSO-d₆, and 3.34 ppm for CD₃OD). Spin multiplicities are described as s (singlet), brs (broad singlet), d (doublet), t (triplet) and m (multiplet). Total ion current traces were obtained for electrospray positive and negative ionization (ES+/ES-) on a Bruker Esquire Liquid Chromatograph-Ion trap mass spectrometer. Analytical chromatographic conditions used for the LC/MS analysis: Column, Zorbax™ Extend C18 from Agilent technologies, 2.1x100 mm. The stationary phase particle size is 3.5µM. Solvents were A, aqueous solvent = water +5% acetonitrile+1% acetic acid; B, organic solvent = acetonitrile + 1% acetic acid; Methods, 14 min run time (0-10 min 20-100% B, flow rate-0.275 mL/min; 10-12 min 100% B, flow rate-0.350 mL/min; 12-12.50 min 100-20% B, flow rate-0.350 mL/min; 12.50-14.0 min 20% B, flow rate-0.350 mL/min). The following additional parameters were used: injection volume (10 µL), column temperature (30°C), UV wavelength range (254-330 nm). The purity of all tested compounds was ≥ 95% using the analytical method described above unless stated otherwise. Analytical HPLC analyses were performed on a Supelco SupelcoSIL™ LC18 column (5 µm, 4.6mm x 25cm) with a linear elution gradient ranging from 0-100% ACN over 27 min, using a SupelcoSIL™ LC18 column (5 µm, 4.6mm x 25cm) at a flow rate of 1 mL/min. A purity of>95% has been established for all tested compounds.

For Compound 3, reagents and starting materials were similarly obtained from commercial sources and similar analytical methods and criteria used. For Compound 1 alternative final step and for Compound 3, the NMR spectrometer used for ¹H NMR was an Avance™ III Bruker instrument operating at 400.31 MHz and for liquid chromatography mass spectrometry an Agilent LCMS system with mass detector was used.

### Example 1: Synthesis of compounds according to the invention

The triazolopyrimidine derivatives can be prepared from readily available starting materials using methods and procedures known from the skilled person. It will be appreciated that where typical or preferred experimental conditions (i.e. reaction temperatures, time, moles of reagents, solvents etc.) are given, other experimental conditions can also be used unless otherwise stated. Optimum reaction conditions may vary with the particular reactants or solvents used, but such conditions can be determined by the person skilled in the art, using routine optimisation procedures. The title compounds of the invention are synthesized as described in the general synthetic route, Scheme1 below.

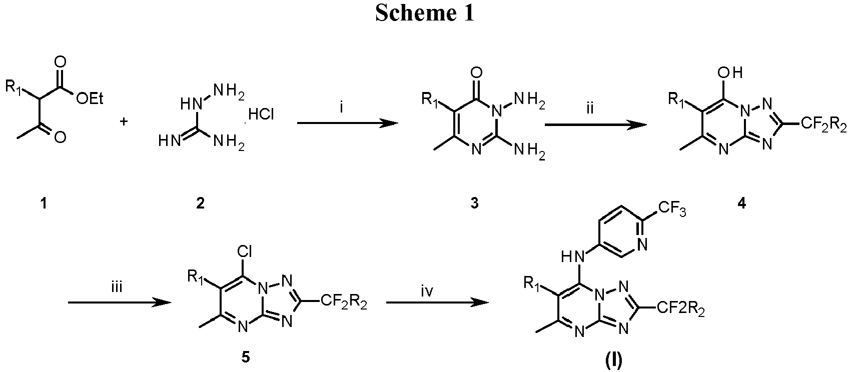

Reagents and Conditions: **i)** a) NaOH, EtOH, 3h at RT; (or) b) NaOEt, EtOH, reflux 5 h, overnight at RT; **ii)** Appropriate difluoro ester, e.g. ethyl 2,2 difluoro propionate, NaOEt, reflux, 4h; **iii**) reflux in POCl₃, 1h; **iv)** 5-Amino-2-trifluoromethylpyridine, EtOH, 5-8 h, RT or 50°C.

### 2-(1,1-difluoroethyl)-5-methyl-N-(6-(trifluoromethyl)pyridin-3-yl)-[1,2,4]triazolo[1,5-a]pyrimidin-7-amine (Compound (1))

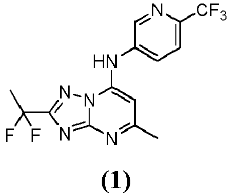

The title compound of the invention was synthesized as described in Scheme 1 above wherein intermediate 1 is such that R₁ is H (1**a**), intermediate 3 wherein R₁ is H **(3a),** intermediate 4 wherein R₁ is H and R₂ is CH₃ **(4a)** and the same applies to intermediate 5 **(5a)** under the same reaction conditions.

### Compound (3a)

To a solution of aminoguanidine hydrochloride **(2)** (11.05 g, 99.95 mmol) in 40 ml of water were sequentially added drop wise a solution of sodium hydroxide (8 g, 200 mmol) in 10 ml of water and ethyl acetoacetate (**1a**) (18.9 ml, 150 mmol) in 15 ml of ethanol, under vigorous stirring. The mixture was stirred for 3 h. The precipitate obtained was filtered and dried under vacuum to afford intermediate **3a** 2,3-diamino-6-methyl-4(3H)-pyrimidinone, which was used without purification for subsequent steps. Alternatively, compound 3a can be obtained as follows: To a solution of NaOEt prepared from sodium (9.19 g, 400 mmol) and ethanol (350 mL), aminoguanidine hydrochloride **(2)** (44.2 g, 400 mmol) was added and the reaction was heated at 50°C for 30 min. Then, the reaction was filtered to remove NaCl and ethyl acetoacetate (**1a**) (25.29 ml, 200 mmol) was added to the filtrate, the reaction mixture was heated at reflux for 5 h, and then stirred at RT overnight. The precipitate obtained was filtered and dried under vacuum to afford intermediate **3a** 2,3-diamino-6-methyl-4(3H)-pyrimidinone.

### Compound (4a)

To a solution of NaOEt prepared from sodium (3.3g, 143 mmol) and ethanol (150 mL), intermediate **3a** (10.0 g, 71.4 mmol) was added, and the reaction mixture was heated at 80°C for 30 min. Then, the reaction mixture was cooled down to room temperature, and ethyl 2,2-difluoropropanoate (10.8ml, 85.68mmol) was added. The mixture was stirred at room temperature for 30 min before being heated to 80°C for 3 h. The reaction mixture was concentrated to dryness, and water (200 mL) was added. The reaction mixture pH was adjusted to 4 by addition of 2N aq HCl solution while a white solid precipitated. The solid was filtered off, washed with water, and dried under vacuum to afford 2-(1,1-difluoroethyl)-5-methyl-[1,2,4]triazolo[1,5-a]pyrimidin-7-ol **(4a):** ¹H NMR (300 MHz, CD₃OD): δ ppm: 13.43 (brs, 1H), 5.92 (s, 1H), 2.33 (s, 3H), 2.08 *(t, J=* 19.2 Hz, 3H). ESIMS *m*/*z:* 215 (MH)⁺.

### Compound (5a)

A suspension of intermediate **4a** (0.25 g, 1.17 mmol) in phosphorus oxychloride (0.33 ml, 3.5 mmol) was heated at reflux for 1 h. The reaction mixture was added drop wise into iced water, neutralized with solid Na₂CO₃, and product was extracted with DCM. The combined organic layers were washed with brine and dried over anhydrous Na₂SO₄. Brown oil was obtained upon solvent *removal in vacuo,* which was purified by flash chromatography on silica gel, eluting with hexanes: EtOAc mixtures to yield 7-Chloro-2-(1,1-difluoroethyl)-5-methyl[1,2,4]triazolo[1,5-a] pyrimidine **(5a).** ¹H NMR (300 MHz, CDCl₃) δ (ppm): 7.17 (s, 1H), 2.75 (s, 3H), 2.18 (t, *J=* 18.7 Hz, 3H). ESIMS *m*/*z* 233 (MH)⁺.

### Compound 1

To a suspension of intermediate **5a** (0.23 g, 1 mmol) in ethanol (5 mL), 5-Amino-2-trifluoromethylpyridine (0.16 g, 1 mmol) was added and the mixture was stirred at RT or at 50°C until the reaction reached completion. Ammonia solution 7N in methanol (50 µl) was added and solvent was removed in vacuo and the crude mixture was purified by flash chromatography (silica gel, eluting with hexane:EtOAc mixtures from 75:25 to 25:75%) to yield the title compound 1 2-(1,1-difluoroethyl)-5-methyl-N-(6-(trifluoromethyl)pyridin-3-yl)-[1,2,4]triazolo[1,5-a]pyrimidin-7-amine. ¹H NMR (300 MHz, CD₃OD) δ (ppm): 8.86 (s, 1H), 8.17(d, J= 8.2 Hz, 1H), 7.96 (d, *J=* 8.5 Hz, 1H), 6.75 (s, 1H), 2.57 (s, 3H), 2.16 *(t, J=* 18.85 Hz, 3H). ESIMS *m*/*z* : 359.4 (MH)⁺.

An alternative final step for Compound 1 is detailed in Scheme 2 below.

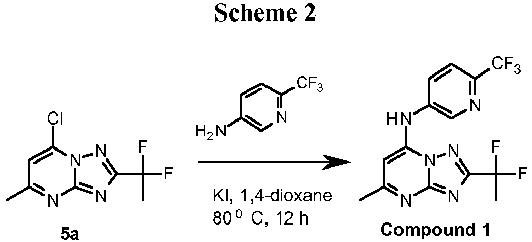

To a stirred solution of 7-chloro-2-(1,1-difluoroethyl)-5-methyl-[1,2,4]triazolo[1,5-a]pyrimidine obtained as described above (3.0 g, 0.013 mol) in 1, 4-dioxane (30 mL) were added 6-(trifluoromethyl) pyridin-3-amine (2.1 g, 0.13 mol) followed by KI (0.42 g, 0.002 mol). The reaction mixture was heated to 80° C for 12 h. After the reaction was complete, the reaction mixture was concentrated under reduced pressure, and the crude product was dissolved in ethyl acetate and washed with 10% NaHCO₃ solution, water, brine and dried over anhydrous Na₂SO₄. Solvent was removed under reduced pressure to get the crude product which was purified by column chromatography to obtain pure 2-(1,1-difluoroethyl)-5-methyl-N-(6-(trifluoromethyl)pyridin-3-yl)-[1,2,4]triazolo[1,5-a]pyrimidin-7-amine **(compound 1)** (3.2 g, 69% yield). **LCMS** (m/z): 359.2 (M⁺). **¹H NMR** (400 MHz, CD₃OD): δ 8.84 (s, 1H), 8.16 (d, *J*=7.6Hz, 1H), 7.94 (d, *J*=8.4Hz, 1H), 6.74(s, 1H, NH), 2.55 (s, 3H), 2.15 (t, *J*=18.8Hz, 3H).

### 2-(1,1-difluoroethyl)-6-fluoro-5-methyl-N-(6-(trifluoromethyl)pyridin-3-yl)-[1,2,4] triazolo[1,5-a]pyrimidin-7-amine (Compound (2))

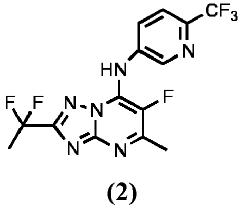

The title compound of the invention was synthesized as described in Scheme 1 above wherein intermediate 1 is such that R₁ is F **(1b),** intermediate 3 wherein R₁ is F **(3b),** intermediate 4 wherein R₁ is F and R₂ is CH₃ **(4b)** and the same applied to intermediate 5 **(5a)** under the same reaction conditions.

### Compound (3b)

To a solution of aminoguanidine hydrochloride **(2)** (11.05 g, 99.95 mmol) in 40 ml of water were sequentially added drop wise a solution of sodium hydroxide (8 g, 200 mmol) in 10 ml of water and ethyl 2-fluoroacetoacetate (**1b**) (18.8 ml, 150 mmol) in 15 ml of ethanol, under vigorous stirring. The mixture was stirred for 3 h. The precipitate obtained was filtered and dried under vacuum to afford intermediate 2,3-diamino-5-fluoro-6-methyl-4(3H)-pyrimidinone **(3b),** which was used without purification for subsequent steps. Alternatively, compound 3b was made as follows: To a solution of NaOEt prepared from sodium (9.19 g, 400 mmol) and ethanol (350 mL), aminoguanidine hydrochloride **(2)** (44.2 g, 400 mmol) was added and the reaction was heated at 50°C for 30 min. Then, the reaction was filtered to remove NaCl and ethyl 2-fluoroacetoacetate (**1b**) (25 ml, 200 mmol) was added to the filtrate, the reaction mixture was heated at reflux for 5 h, and then stirred at RT overnight. The precipitate obtained was filtered and dried under vacuum to afford intermediate 2,3-diamino-5-fluoro-6-methyl-4(3H)-pyrimidinone **(3b).**

### Compound (4b)

To a solution of NaOEt prepared from sodium (3.3g, 143 mmol) and ethanol (150 mL), intermediate **3b** (11.28g, 71.4 mmol) was added, and the reaction mixture was heated at 80°C for 30 min. Then, the reaction mixture was cooled down to room temperature, and ethyl 2,2-difluoropropanoate (10.8 ml, 85.68 mmol) was added. The mixture was stirred at room temperature for 30 min before being heated to 80°C for 3 h. The reaction mixture was concentrated to dryness, and water (200 mL) was added. The reaction mixture pH was adjusted to 4 by addition of 2N aq. HCl solution while a white solid precipitated. The solid was filtered off, washed with water, and dried under vacuum to afford intermediate 2-(1,1-difluoroethyl)-6-fluoro-5-methyl-[1,2,4]triazolo[1,5-a]pyrimidin-7-ol **(4b):** ¹H NMR (300 MHz, CD₃OD) δ (ppm): 2.63 (d, *J =* 3.72 Hz, 3H), 2.26 (t*, J =* 19.07 Hz, 3H). ESIMS *m*/*z:* 233.4 (MH)⁺.

### Compound 5b

A suspension of intermediate **4b** (0.27g, 1.17 mmol) in phosphorus oxychloride (0.33 ml, 3.5 mmol) was heated at reflux for 1 h. The reaction mixture was added drop wise into iced water, neutralized with solid Na₂CO₃, and product was extracted with DCM. The combined organic layers were washed with brine and dried over anhydrous Na₂SO₄. Brown oil was obtained upon solvent removal in vacuo, which was purified by flash chromatography on silica gel, eluting with hexanes: EtOAc mixtures to yield 7-chloro-2-(1,1-difluoroethyl)-6-fluoro-5-methyl-[1,2,4]triazolo[1,5-a]pyrimidine **(5b).** ¹H NMR (300 MHz, CDCl₃): δ ppm: 2.60 (d, *J=* 3.8 Hz, 3H), 2.10 (t, *J=* 19 Hz, 3H), ESIMS *m*/*z:* 251.3 (MH)⁺.

### Compound 2

To a suspension of intermediate **5b** (0.25 g, 1 mmol) in ethanol (5 mL), 5-Amino-2-trifluoromethylpyridine (0.16 g, 1 mmol) was added and the mixture was stirred at RT or at 50 °C until the reaction reached completion. Ammonia solution 7N in methanol (50 µl) was added and solvent was *removed in vacuo* and the crude mixture was purified by flash chromatography (silica gel, eluting with hexane:EtOAc mixtures from 75:25 to 25:75%) to yield Compound 2, **6-fluoro-2-(1,1-difluoroethyl)-N-(6-(trifluoromethyl)pyridin-3-yl)-5-methyl-[1,2,4]triazolo[1,5-a]pyrimidin-7-amine.** ¹H NMR (300 MHz, CDCl₃) δ (ppm): 8.69 (s, 1H), 7.83 (brs, 1H), 7.8-7.66 (m, 2H), 2.65 *(d, J=* 3.84 Hz, 3H), 2.14 (t, *J*= 18.68 Hz, 3H). ESIMS *m*/*z* : 377.1 (MH)⁺.

### 5-methyl-2-(trifluoromethyl)-N-(6-(trifluoromethyl)pyridin-3-yl)-[1,2,4]triazolo[1,5-a] pyrimidin-7-amine (Compound (3))

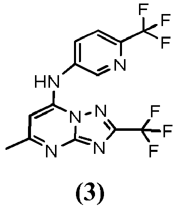

The title compound of the invention was synthesized by an alternative general route as exemplified in Scheme 3 below:

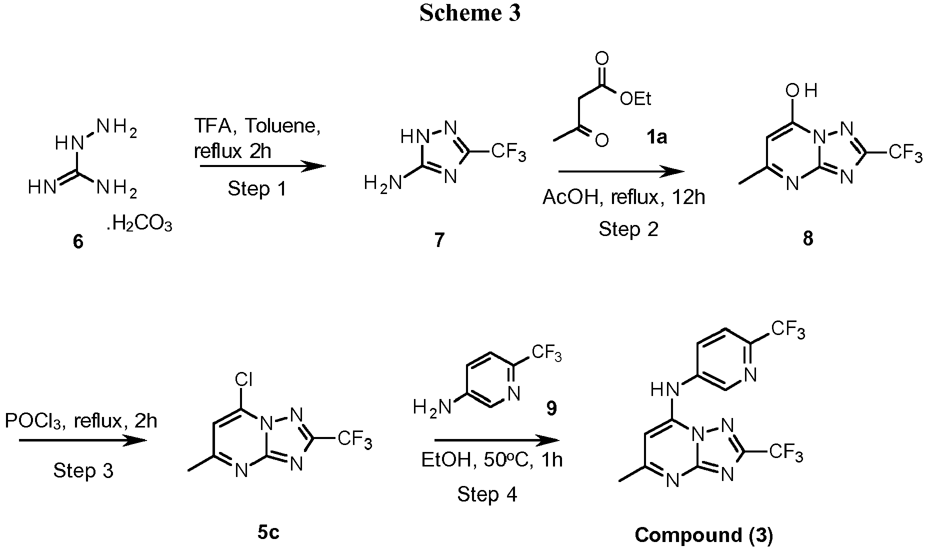

**Step 1:** Amino guanidine bicarbonate **(6)** (2.0 g, 15.0 mmol) and trifluoro acetic acid (10 mL) were stirred at 25°C till effervescence ceased. Toluene was added to the reaction mixture and the reaction mixture refluxed for 20 h using a Dean and Stark condenser. The reaction mixture was cooled, and the solid precipitate was filtered and dried to yield product 7, 3-(trifluoromethyl)-1H-1,2,4-triazol-5-amine (1.0 g, 45.4%).

**Step 2:** To a slurry of 7 (1.0 g, 6.5 mmol) in acetic acid (20 mL) was added ethyl acetoacetate **(Ia)** (3.4 g, 6.5 mmol) and the reaction mixture heated to reflux for 12 h. The reaction mixture was cooled to 25°C and the acetic acid was removed under reduced pressure. The crude compound was triturated with dichloromethane to yield **8,** 5-methyl-2-(trifluoromethyl)-[1,2,4]triazolo[1,5-a]pyrimidin-7-ol (1.2 g, 85.1%). **¹H NMR** (400 MHz, DMSO D6 + D20): δ 5.94 (s, 1H), 2.32 (s, 3H).

**Step 3:** Phosphoryl chloride (5 mL) was added to **8** (0.3 g, 13.7 mmol) and the reaction mixture was heated to reflux for 2 h. The reaction mixture was cooled to 25°C and phosphoryl chloride was removed under reduced pressure. Crude product was quenched with crushed ice and neutralized with 10% NaHCO₃ solution and extracted with ethyl acetate (2 x 5 mL). The combined organic layers were washed with brine solution, dried over Na₂SO₄ and concentrated under reduced pressure. Crude product was purified by column chromatography using 15-25% pet. ether and ethyl acetate to yield compound **5c,** 7-Chloro-5-methyl-2-(trifluoromethyl) -[1,2,4]triazolo[1,5-a] pyrimidine (0.17 g, 53.1%). **¹H NMR** (400 MHz, CDCl₃): δ 7.27 (s, 1H), 2.78 (s, 3H).

**Step 4:** To a solution of **5c** (0.1 g, 4.2 mmol) in ethanol (6 mL) was added 5-amino-2-trifluoromethyl pyridine **(9)** (0.068 g, 4.2 mmol) and the resulting mixture heated at 50°C for 1 h. The reaction mixture was cooled to 25°C and solvent removed under reduced pressure. Crude product was dissolved in dichloromethane and washed with 10% NaHCO₃ solution and brine solution. The organic layer was dried over Na₂SO₄, filtered and concentrated. Crude product was purified by column chromatography using 20-40% pet. ether and ethyl acetate to yield Compound 3, 5-methyl-2-(trifluoromethyl)-N-(6-(trifluoromethyl)pyridin-3-yl)-[1,2,4]triazolo[1,5-a]pyrimidin-7-amine (0.064 g, 42%). **LC-MS APCI:** Calculated for C13H8F6N6 362.24; Observed *m*/*z* [M+H]⁺ 363.2. Purity by LC-MS: 99.05 %. RT: 2.57. **LCMS** (m/z): 363.2 (M⁺). **¹H NMR** (400 MHz, MeOD): δ 8.86 (s, 1H), 8.17 (d, *J=* 7.00 Hz, 1H), 7.97 (d, J= 8.48 Hz, 1H), 6.81 (s, 1H), 2.59 (s, 3H).

If the above synthetic methods are not applicable to obtain triazolopyrimidine derivatives according to the invention and/or necessary intermediates, suitable methods of preparation known by a person skilled in the art should be used. In general, the synthesis pathways for any individual derivative will depend on the specific substituents of each molecule and upon the ready availability of intermediates necessary; again such factors being appreciated by those of ordinary skill in the art. For all the protection and deprotection methods, see Philip J. Kocienski, in "Protecting Group ", Georg Thieme Verlag Stuttgart, 2005 and Theodora W. Greene and Peter G. M. Wuts in "Protective Groups in Organic Synthesis", Wiley Interscience, 4th Edition 2006*.* Compounds of this invention can be isolated in association with solvent molecules by crystallization from evaporation of an appropriate solvent. The pharmaceutically acceptable acid addition salts of the triazolopyrimidine derivatives, may be prepared in a conventional manner. For example, a solution of the free base may be treated with a suitable acid, either neat or in a suitable solution, and the resulting salt isolated either by filtration or by evaporation under vacuum of the reaction solvent. Pharmaceutically acceptable base addition salts may be obtained in an analogous manner by treating a solution of a triazolopyrimidine derivative with a suitable base. Both types of salts may be formed or interconverted using ion-exchange resin techniques.

### Example 2: Antimalarial activities of compounds of the invention

The ability of triazolopyrimidine derivatives according to the invention to kill *P. falciparum* parasites and/or to inhibit its proliferation is assayed through their ability to inhibit *Plasmodium falciparum* growth. The growth inhibition assay is as follows: *P. falciparum* 3D7 cells were grown in Gibco-Invitrogen RPMI-1640 supplemented with 2% (w/v) red blood cells (RBCs) and either 20% human type A+ plasma (Desjardins et al., 1979, Antimcrob Agents Chemother. 16, 710-718*)* or with Gibco-Invitrogen 0.5% Albumax I (Coteron et al., 2011, J. Med. Chem., 54, 5540-5561*).* Serial dilutions of compound stocks were prepared in 100% DMSO at 200X the final concentration, followed by generation of 20X stocks in media. Cell growth was monitored by the SYBR green method as described *(*Deng, et al., 2014. J. Med .Chem., 57, 5381-539*).* Parasites (0.19 ml of 0.5% parasitemia, 0.5% HCT) were plated into 96-well microtiter plates containing 10 µL compound or DMSO control (final DMSO concentration of 0.5%). Non-parasitized RBCs (0.5% HCT) were used as a control to determine background fluorescence. After 72 h of incubation parasitized RBCs were quantitated by the SYBR Green method. 2X SYBR Green I solution (20 µL) in 1X PBS was mixed with 20 µL parasites in 96-well plates, incubated for 20 min, and after which 160 µL of 1X PBS was added. Fluorescence was detected using a BD Biosciences Acurri C6 flow cytometer and events recorded within gates that encompassed all asexual growth stages of the *P. falciparum* intraerythrocytic life cycle. A minimum 50,000 total events were recorded per well. Background events determined from non-parasitized RBC controls were subtracted from final counts. Data at each concentration point were collected in triplicate and were fitted to the log[I] vs response - variable slope (4 parameter) model in Graph Pad Prism to determine the concentration of inhibitor that resulted in 50% growth inhibition (ED₅₀).

The compounds of the invention were also assessed for inhibitory activity against recombinant DHODH from the indicated species. Recombinant enzymes were produced and purified from *E. coli* as His₆ fusion proteins and purified by Ni⁺²-agarose chromatography as described. Steady-state kinetic assays to determine inhibitor IC₅₀'s were performed with a dye-based assay that couples the final oxidation of CoQ to the reduction of 2,6-dichloroindophenol (DCIP) followed at 600 nm (e=18.8 mM⁻¹cm⁻¹) as described (Baldwin et al., 2002, J. Biol. Chem., 277,41827-41834 and Deng et al,, 2014. J. Med. Chem., 57, 5381-539*).*

Data were collected on a Synergy H1 hybrid plate reader in 96 well plate format at 28°C, using a final assay volume of 200 µl. Rate data (v) were converted to µmoles/min using the molar extinction coefficient for DCIP. Compounds were prepared in stock solutions of DMSO. An initial 100 mM stock was generated and this was used to prepare a 3 fold dilution series in DMSO. A 1:100 dilution of DMSO stock was made into assay buffer to generate the final concentration in the assay mix.

*Assay Conditions:* DHODH (E_{T} = 5-10 nM), substrates (0.2 mM L-dihydroorotate and 0.02 mM CoQ_{D}), DCIP (0.12 mM) and assay buffer (100 mM HEPES, pH 8.0, 150 mM NaCl, 10% Glycerol, 0.1%Triton) at 20°C. Concentration of compounds of the invention was varied in a 3-fold dilution series (0.01 - 100 uM). All data were collected in triplicate.

The percent inhibition relative to the no inhibitor control was determined (vᵢvₒ x100) and data were fitted to either the log[I] vs response (three parameters) equation (Y=Bottom + (Top-Bottom)/(1+10^((X-LogIC₅₀)))) or for compounds of the invention with IC₅₀ > 10 uM data were fitted to the standard IC₅₀ equation (Y=Ymax/(1+(X/IC₅₀))). Fitting was performed in Graph Pad Prism to determine the IC₅₀. Reported error represents the 95% confidence interval of the fit or the standard deviation of the mean. Activities against 3D7, pfDHODH, human DHODH(h), mouse DHODH (m), rat DHODH (r) and dog DHODH](d) are reported in Table 1 below. The antimalarial activities of compounds of the invention have been compared to other triazolopyrimidines which have been said to show some inhibitory activities against *Plasmodium falciparum* (WO 2011/041304) and some compounds having closely related structures.

### Example 3: Aqueous solubility of compounds of the invention

Aqueous solubility was estimated by nephelometry. Concentrated stock solutions were prepared in DMSO and diluted into either pH 6.5 phosphate buffer or 0.01 M HCl (approximately pH 2.0), with the final DMSO concentration being 1%. Samples were then analyzed by nephelometry to determine the solubility range as described previously *(*Bevan, et al., 2000, Anal. Chem., 72, 1781-1787*).* The solubility results are presented in Table 1 below.

**Table 1**

| **Compound** | **Pf 3D7 EC₅₀ (nM)** | **P*f*DHODH (nM)** | **h/d/m/r DHODH (µM)** | **Kinetic Solubility pH6.5 (µg/ml)** |
|---|---|---|---|---|
| 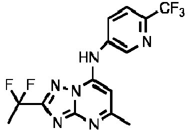 | 11 | 30 | >100,65,54,24 | >100 |
| (1) | | | | |
| 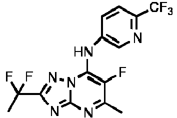 | 16 | 23 | 62, 9.2, 20, 15.7 | >100 |
| (2) | | | | |
| 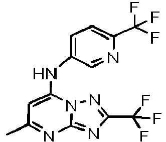 | 35 | 41 | >100, 39, 33, 8.4 | 50 |
| (3) | | | | |
| 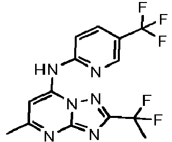 | 20 | 50 | 58, 21, 74, 4.4 | 6.3 |
| Reference 1 | | | | |
| 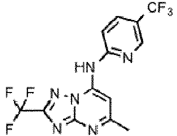 | 70 | 37 | >50,nd,nd,nd | 1.6 |
| Reference 2 | | | | |
| 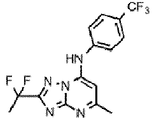 | 8.3 | 31 | >100, >100, 24, 7.2 | 50 |
| Reference 3 | | | | |
| 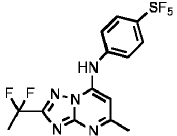 | 5.0 | 33 | >100, 17, 2.7, 2.2 | 25 |
| Reference 4 | | | | |

### Example 4: Anti-malarial in vivo efficacy of compounds according to the invention

The ability of triazolopyrimidine derivatives according to the invention to show antimalarial *efficacy in vivo* using the SCID mouse *P. falciparum* model. *Briefly,* NOD-*scid IL-2Rγnull* (NSG) mice (Jackson Laboratory, USA) (23 - 36 g) engrafted with human erythrocytes as described were infected with 20 x 10⁶ *P. falciparum* Pf3D70087/N9) generated in GlaxoSmithkline Tres Cantos (Spain) by intravenous injection. Compounds were administered orally in vehicle (saline solution for Chloroquine; 0.5% w/v sodium carboxymethylcellulose, 0.5% v/v benzyl alcohol, 0.4% v/v Tween 80 in water for triazolopyrimidine analogs). Compounds were administered at target doses ranging from 0.5 to 75 mg/kg (free base equivalent) starting on day 3 post infection. Formulation concentrations were measured to obtain the actual doses administered. Parasitemia was monitored by flow cytometry and the effective dose (ED₉₀) was calculated as described in Jimenez-Diaz, 2009, Antimicrobial agents and chemotherapy, 53, 4533-4536*.* Results for some compounds of the invention are presented in Table 2 below.

**Table 2**

| **Compound** | **ED₉₀ (mg/kg per day)** |
|---|---|
| (1) | 2.6 |
| Reference 3 | 26 |
| Reference 4 | 8.1 |
| Pyrimethamine | 0.9 |
| Chloroquine | 4.3 |

Altogether, those data support that compounds of the invention have improved or good solubility while preserving in vivo efficacy and good selectivity for the plasmodium enzyme over the mammalian enzymes.

### Example 5: Anti-malarial ex vivo efficacy of compounds according to the invention

To assess comparative activity between *P.falciparum* and *P. vivax* strains compounds were tested in an *ex vivo* parasite assay using *Plasmodium* isolates from patients who visited clinics in Timika (Papua, Indonesia). Parasites in this region are resistant to standard anti-malarials such as chloroquine. *P. vivax* can not be cultivated *in vitro* so the *ex vivo* assay is the only method to evaluate drug sensitivity. Patients with parasitaemia between 2000 and 80,000 ul⁻¹ were eligible for recruitment into the study. Blood was collected by venipuncture, host white blood cells were removed and packed infected red blood cells were used for the *ex vivo* studies. Drug susceptibility was measured as previously described (Russell et al., 2008, Antimicrobial Agents Chemother., 52, 1040-1045*;* Marfurt et al, 2011, Antimicrobial Agents Chemother., 55, 961-966*;* Marfurt et al., 2011, Antimicrobial Agents Chemother., 55, 4461-4464*).* Briefly two hundred µl of a 2% hematocrit blood mixture, consisting of RPMI 1640 medium plus 10% AB+ human serum *(P. falciparum*) or McCoy's 5A medium plus 20% AB+ human serum *(P. vivax)* was added to each well of pre-dosed drug plates containing serial 2-fold dilutions of the anti-malarial compound. A candle jar was used to mature parasites at 37°C for 35-56 hrs. Incubation was stopped when 40% of ring stage parasites had reached mature schizont stage in drug-free control wells. Thick blood films made from each well were stained with 5% Giemsa for 30 min and examined microscopically. The number of schizonts per 200 asexual stage parasites was determined and normalized to the control well. Data were analyzed by nonlinear regression (WinNonLn 4.1) to determine the IC₅₀ using the inhibitory sigmoid Eₘₐₓ model. In this model, Compound 1 shows equivalent activity on both *P.falciparum* and *P. vivax* whereas compound of Reference 4 does not, and thus might be expected to require higher doses for the treatment of *P. vivax.* Note in this assay the IC₅₀ for DHODH inhibitors is higher than standard 72 hr proliferation assays and this is thought to be a reflection of the shorter assay time. Results are presented in Table 3 below.

**Table 3**

| **Compounds** | ***P*. *falciparum* FC27 lab lines IC₅₀ (nM)** | **P*.falciparum* clinical isolates IC₅₀ (nM)** | **P. *vivax* clinical isolates IC₅₀ (nM)** |
|---|---|---|---|
| (1) | 292 | 151 | 175 |
| Reference 4 | 177 | 190 | 920 |
| Chloroquine | 23-32 | 86 - 101 | 43 - 147 |
| Artesunate | 3.6 - 11 | 2.4 - 2.4 | 0.8 - 2.9 |

FC27 is a chloroquine sensitive lab strain.

### Ouality control (QC) procedures

Drug plate quality was assured by running schizont maturation assays (two independent experiments) with the chloroquine-resistant strain K1 and the chloroquine-sensitive strain FC27. For microscopy slide reading QC, two randomly selected drugs per isolate were read by a second microscopist. If the raw data derived by the two microscopists lead to a dramatic shift in the IC50 estimates of the selected drugs, the whole assay (i.e., all standard drugs and experimental compounds) was re-read by a second reader and the results compared. If necessary, discrepant results were resolved by a third reading by an expert microscopist.

## Claims

1. A triazolopyrimidine derivative according to Formula (I), 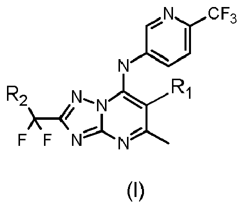 wherein R₁ is selected from halogen and H, R₂ is selected from F and C1-C4 alkyl; as well as any pharmaceutically acceptable salt, hydrate, solvate, polymorph, tautomers, geometrical isomers, or optically active isomers thereof.

2. A triazolopyrimidine derivative according to claim 1, wherein R₁ is H.

3. A triazolopyrimidine derivative according to claim 1, wherein R₁ is F.

4. A triazolopyrimidine derivative according to any one of claims 1 to 3, wherein R₂ is CH₃.

5. A triazolopyrimidine derivative according to any one of claims 1 to 3, wherein R₂ is F.

6. A triazolopyrimidine derivative according to any one of claims 1 to 5, selected from the following group:
2-(1,1-difluoroethyl)-5-methyl-N-(6-(trifluoromethyl)pyridin-3-yl)-[1,2,4]triazolo[1,5-a]pyrimidin-7-amine;
2-(1,1-difluoroethyl)-6-fluoro-5-methyl-N-(6-(trifluoromethyl)pyridin-3-yl)-[1,2,4]triazolo[1,5-a]pyrimidin-7-amine; and
5-methyl-2-(trifluoromethyl)-N-(6-(trifluoromethyl)pyridin-3-yl)-[1,2,4]triazolo[1,5-a]pyrimidin-7-amine; as well pharmaceutically acceptable salt, hydrate, solvate, polymorph, tautomers, geometrical isomers, or optically active isomers thereof.

7. A triazolopyrimidine derivative according to any one of claims 1 to 6 for use as a medicament.

8. A pharmaceutical composition comprising at least one triazolopyrimidine derivative according any of claims 1 to 6 or a pharmaceutically acceptable salt thereof, and a pharmaceutically acceptable carrier, diluent or excipient thereof.

9. A pharmaceutical composition according to claim 8 further comprising an antimalarial co-agent.

10. A pharmaceutical composition according to claim 9 wherein the co-agent is selected from artemisinin or an artemisinin and its derivatives (such as artemether or dihydroartemisinin, chloroquine, quinine, mefloquine, amodiaquine, atovaquone/proguanil, doxycycline, lumefantrine, piperaquine, pyronaridine, halofantrine, pyrimethamine-sulfadoxine, primaquine, quinacrine, doxycycline, atovaquone, proguanil hydrochloride, piperaquine, ferroquine, tafenoquine, arterolane, Spiro[3H-indole-3,1'-[1H]pyrido[3,4-b]indol]-2(1H)-one, 5,7'-dichloro-6'-fluoro-2',3',4',9'-tetrahydro-3'-methyl-,(1'R,3'S)- (CAS Registry Number: 1193314-23-6), Sulfur, [4-[[2-(1,1-difluoroethyl)-5-methyl[1,2,4]triazolo[1,5-a]pyrimidin-7-yl]amino]phenyl] pentafluoro-] (CAS Registry Number: 1282041-94-4), Morpholine,4-[2-(4-cis-dispiro[cyclohexane-1,3'-[1,2,4]trioxolane-5',2"-tricyclo [3.3.1.13'7]decan]-4-ylphenoxy)ethyl]- (CAS Registry Number: 1029939-86-3), [3,3'-Bipyridin]-2-amine, 5-[4-(methylsulfonyl)phenyl]-6'-(trifluoromethyl)- (CAS Registry Number: 1314883-11-8), Ethanone, 2-amino-1-[2-(4-fluorophenyl)-3-[(4-fluorophenyl)amino]-5,6-dihydroimidazo[1,2-a]pyrazin-7(8H)-yl]- (CAS Registry Number 1261109-90-3).

11. A triazolopyrimidine derivative according to any one of claims 1 to 6 for use in the prevention or treatment of malaria.

12. A method for preparing a compound of Formula (I) wherein R₁ is F and R₂ is CH₃ comprising a step of reacting a triazolopyrimidine of Formula (5b) in presence of 5-amino-2-trifluoromethylpyridine as follows:

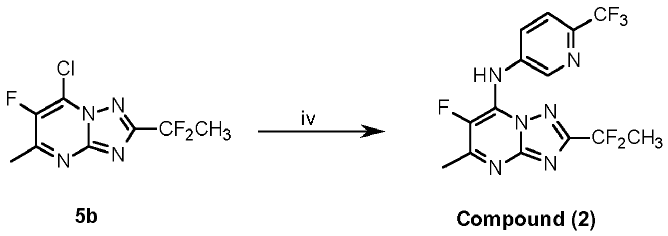

13. A method for preparing a compound of Formula (I) wherein R₁ is F and R₂ is CH₃ comprising a step of reacting a triazolopyrimidine of Formula (4b) in presence of POCl₃ to lead to an intermediate of Formula (5b) as follows:

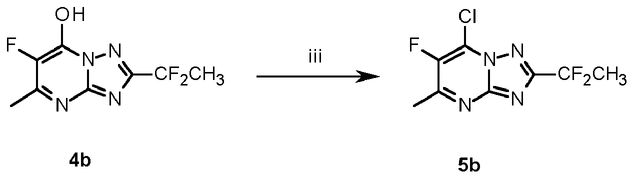

14. An intermediate wherein the intermediate is 2-(1,1-difluoroethyl)-6-fluoro-5-methyl-[1,2,4]triazolo[1,5-a]pyrimidin-7-ol.

15. An intermediate wherein the intermediate is 7-chloro-2-(1,1-difluoroethyl)-6-fluoro-5-methyl-[1,2,4]triazolo[1,5-a]pyrimidine.
